(19) Europäisches Patentamt
European Patent Office
Office européen des brevets

(11) **EP 3 771 449 A1**

(12) **EUROPÄISCHE PATENTANMELDUNG**

(43) Veröffentlichungstag:
**03.02.2021 Patentblatt 2021/05**

(51) Int Cl.:
***A61B 34/10*** (2016.01)

(21) Anmeldenummer: **19189356.9**

(22) Anmeldetag: **31.07.2019**

(84) Benannte Vertragsstaaten:
**AL AT BE BG CH CY CZ DE DK EE ES FI FR GB
GR HR HU IE IS IT LI LT LU LV MC MK MT NL NO
PL PT RO RS SE SI SK SM TR**
Benannte Erstreckungsstaaten:
**BA ME**
Benannte Validierungsstaaten:
**KH MA MD TN**

(71) Anmelder:
• **Siemens Healthcare GmbH
91052 Erlangen (DE)**

• **Friedrich-Alexander-Universität
Erlangen-Nürnberg
91054 Erlangen (DE)**

(72) Erfinder:
• **Breininger, Katharina
91052 Erlangen (DE)**
• **Pfister, Marcus
91088 Bubenreuth (DE)**

(54) **VERFAHREN ZUR VERFORMUNGSSIMULATION UND VORRICHTUNG**

(57) Verfahren zur Verformungssimulation eines durch das Einführen eines medizinischen Instruments verformbaren Hohlorgans mit den folgenden Schritten:
• Bereitstellung eines vortrainierten maschinenlernenden Algorithmus,
• Bereitstellung einer medizinischen 3D-Aufnahme des Hohlorgans mit umgebendem Gewebe, welche 3D-Aufnahme vor einer Einführung eines medizinischen Instruments aufgenommen wurde,
• Segmentierung oder Bereitstellung einer Segmentierung der medizinischen 3D-Aufnahme des Hohlorgans und Ermittlung oder Bereitstellung eines dreidimensionalen Modelles des Hohlorgans,
• Bereitstellung von Informationen über ein einzuführendes medizinisches Instrument, und
• Simulation der durch Einführung des Instruments zu erwartenden Verformung des Hohlorgans auf der Basis der segmentierten medizinischen 3D-Aufnahme des Hohlorgans und des umgebenden Gewebes und der Informationen über das Instrument durch Verwendung des vortrainierten maschinenlernenden Algorithmus.

FIG 5

**EP 3 771 449 A1**

**Beschreibung**

Die Erfindung betrifft ein Verfahren zur Verformungssimulation eines durch das Einführen eines medizinischen Instruments verformbaren Hohlorgans gemäß dem Patentanspruch 1 sowie eine Vorrichtung zur Durchführung eines derartigen Verfahrens gemäß dem Patentanspruch 18.

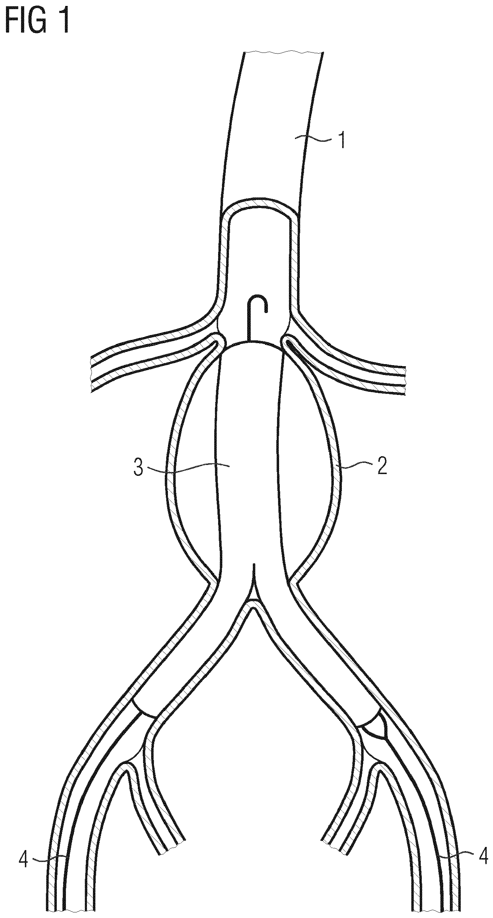

Dies ist besonders relevant auf dem Gebiet der interventionellen Behandlung (z.B. Reparatur) von Aorten-Aneurysmen. Hierbei ist speziell eine nichtlineare Anpassung der Gefäße an eingebrachte steife Instrumente (wie Führungsdrähte, Katheter und Stents) betroffen, eine sogenannte Verformungskorrektur. Ein abdominelles Aortenaneurysma 2 - siehe FIG 1 - ist eine Gefäßaussackung an der abdominellen Aorta 1, deren Verlängerung in die Beinarterien wird als Illiacal-Aneurysma bezeichnet. Behandelt wird dies entweder in einer offenen Bauch-OP oder minimalinvasiv durch Einsetzen eines sogenannten Stentgrafts 3. Ein solches Verfahren wird als EVAR = endovascular aneurysm repair bezeichnet. Über die beiden Leisten werden Führungsdrähte 4 und Katheter in die abdominelle Aorta 1 eingebracht, über die ein oder mehrere Stentgrafts 3 (Kombination aus einem Stent und einem künstlichen Blutgefäß) eingebracht werden.

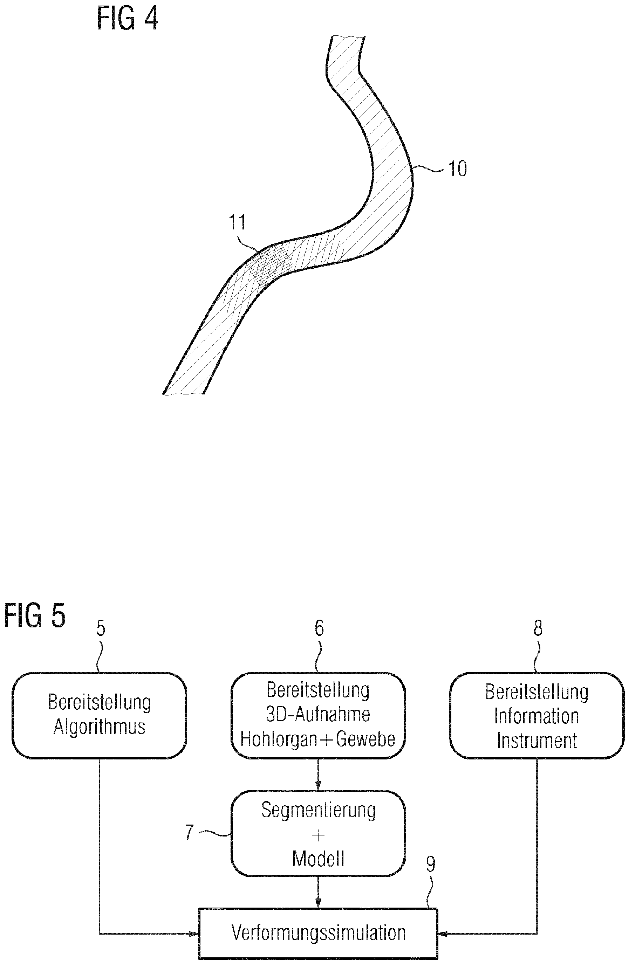

Bei bekannten Verfahren im Stand der Technik werden EVAR Prozeduren auf Angiographiesystemen unter Durchleuchtungskontrolle (Fluoroskopie) durchgeführt. Um dabei die Applikation von jodhaltigem (nierenschädigendem) Kontrastmittel zu minimieren, sind verschiedene Verfahren beschrieben, die dem Fluoroskopiebild registrierte präoperative Datensätze (meistens CT-Angiographien) zu überlagern. Die CTA-Datensätze werden dafür zuvor segmentiert. Üblicherweise treten in den stark gekrümmten Illiacalgefäßen (auch in den Aorten) große Verformungen durch das Einbringen der steifen Instrumente (wie z.B. Führungsdrähte und Katheter) auf. Diese Verformungen hängen prinzipiell von der lokalen Beschaffenheit des Gefäßes und des umliegenden Gewebes ab, sind also nicht homogen. So wird sich das Gefäß beispielsweise in kalzifizierten Bereichen weniger verformen als in anderen Bereichen. In der FIG 4 ist ein Hohlorgan gezeigt, bei dem stark kalzifizierte Regionen 11 eng gestrichelt angezeigt ist.

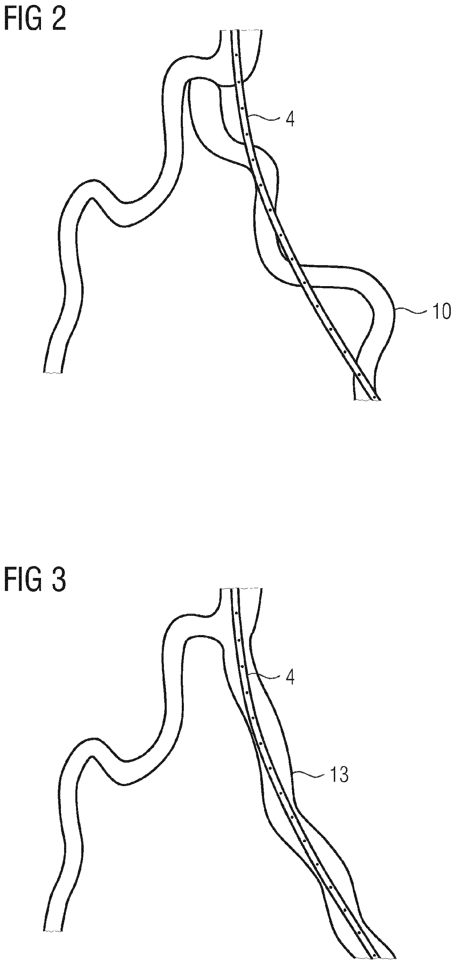

Es existieren Verfahren, diese Verformung in der Überlagerung intra-operativ auszugleichen. So ist z.B. aus dem Artikel von Toth et al., Adaption of 3D Models to 2D X-ray Images during Endovascular Abdominal Aneurysm Repair, Proc. Of the MICCAI Workshop, 2015, pp. 339-346, ein Verfahren zur Bestimmung einer Verformung eines Gefäßes unter Verwendung von überlagerten Bilddatensätzen bekannt. In den Figuren 2 und 3 ist eine derartige intraoperative Verformungskorrektur gezeigt, wobei die FIG 2 das ursprüngliche Hohlorgan 10 sowie einen eingebrachten Führungsdraht 4 in Überblendung zeigt. Die FIG 3 zeigt das korrigierte verformte Hohlorgan 13. Außerdem existieren Verfahren, die die Verformung prä-operativ simulieren, z.B. aus dem Artikel von Roy et al., Finite element analysis of abdominal aortic aneurysms: geometrical and structural reconstruction with application of an anisotropic material model, IMA J Appl Math, 79 (5), 2014, pp. 1011-1026, wobei hier vorbestimmte oder gemessene Eigenschaften von Instrumenten und Gewebe zur Simulation verwendet werden. Dies ist jedoch sehr aufwändig und muss experimentell bestimmt werden.

Es ist Aufgabe der vorliegenden Erfindung, ein Verfahren bereitzustellen, welches eine besonders einfache und robuste Simulation der durch das Einführen eines medizinischen Instruments in ein Hohlorgans bewirkten Verformung gewährleistet; des Weiteren ist es Aufgabe der Erfindung, eine für die Durchführung des Verfahrens geeignete Vorrichtung bereitzustellen.

Die Aufgabe wird erfindungsgemäß gelöst durch ein Verfahren zur Verformungssimulation eines durch das Einführen eines medizinischen Instruments verformbaren Hohlorgans gemäß dem Patentanspruch 1 und von einer Vorrichtung gemäß dem Patentanspruch 18. Vorteilhafte Ausgestaltungen der Erfindung sind jeweils Gegenstand der zugehörigen Unteransprüche.

Bei dem erfindungsgemäßen Verfahren zur Verformungssimulation eines durch das Einführen eines medizinischen Instruments verformbaren Hohlorgans werden die folgenden Schritte durchgeführt: Bereitstellung eines vortrainierten maschinenlernenden Algorithmus, Bereitstellung einer medizinischen 3D-Aufnahme des Hohlorgans mit umgebendem Gewebe, welche 3D-Aufnahme vor einer Einführung eines medizinischen Instruments aufgenommen wurde, Segmentierung oder Bereitstellung einer Segmentierung der medizinischen 3D-Aufnahme des Hohlorgans und Ermittlung oder Bereitstellung eines dreidimensionalen Modelles des Hohlorgans, Bereitstellung von Informationen über ein einzuführendes medizinisches Instrument, und Simulation der durch Einführung des Instruments zu erwartenden Verformung des Hohlorgans auf der Basis der segmentierten medizinischen 3D-Aufnahme des Hohlorgans und des umgebenden Gewebes und der Informationen über das Instrument durch Verwendung des vortrainierten maschinenlernenden Algorithmus.

Durch das erfindungsgemäße Verfahren kann besonders schnell und aufwandsarm automatisch eine besonders realistische und exakte Simulation einer Verformung eines Hohlorgans durch das Einführen eines medizinischen Instruments erstellt werden, indem nicht nur das Hohlorgan selbst sondern auch ganz besonders dessen umgebendes Gewebe einbezogen wird. Insbesondere berücksichtigt der maschinenlernende Algorithmus für die Simulation der Verformung des Hohlorgans Informationen über den Einfluss von Gewebeeigenschaften des umgebenden Gewebes des Hohlorgans. Z.B. Verkalkungen rund um ein Hohlorgan können einen sehr entscheidenden Einfluss auf das Verhalten des Hohlorgans bei der Verformung haben, sie können eine Verformung erschweren. Das erfindungsgemäße Verfahren nutzt diesen Zusammenhang und bezieht das das Hohlorgan umgebende Gewebe in die Verformungssimulation ein. Ein vortrainierter maschinenlernender Algorithmus hat auf diese Weise besonders viele Informationen zur Verfügung, welche einen Einfluss auf die Verformung haben können, so dass eine qualitativ sehr hochwertige Simulation erzielt werden kann. Diese Informationen wiederum helfen einem Arzt signifikant bei der Therapie eines Patienten und bei einer Entscheidung hinsichtlich weiterer Behandlungsschritte, z.B. im Zusammenhang mit der interventionellen Behandlung von Aorten-Aneurysmen.

Nach einer Ausgestaltung der Erfindung wird das Modell des Hohlorgans von einem Oberflächen- oder Volumenmodell gebildet. Derartige Modelle haben sich im Bereich der Segmentierung als besonders vorteilhaft erwiesen.

Nach einer weiteren Ausgestaltung der Erfindung ist der maschinenlernende Algorithmus mittels einer Vielzahl von bekannten Aufnahme-Paaren aus einer ersten medizinischen 3D-Aufnahme eines unverformten Hohlorgans und seines umgebenden Gewebes und einer zweiten medizinischen 3D-Aufnahme eines durch ein medizinisches Instrument verformten Hohlorgans und seines umgebenden Gewebes vortrainiert. Durch möglichst viele, hochqualitative Aufnahme-Paare ist ein besonders effektives Vortraining des Algorithmus möglich, wodurch dann wiederum eine besonders exakte Simulation bewirkt werden kann.

Nach einer weiteren Ausgestaltung der Erfindung sind die Aufnahme-Paare jeweils bezüglich des Hohlorgans segmentiert und das Hohlorgan ist als dreidimensionales Modell mit Teilelementen, insbesondere als Gitternetz mit Gitternetzelementen dargestellt. Eine automatische oder halbautomatische Vorsegmentierung des Hohlorgans ist ein bekanntes Verfahren, welches die weitere Bearbeitung von 2D- oder 3D-Aufnahmen deutlich erleichtert, indem bestimmte Strukturen (also z.B. das Hohlorgan) erkannt und z.B. markiert oder hervorgehoben werden. Anschließend kann das so segmentierte Hohlorgan als Modell aus einer Vielzahl von Teilelementen dargestellt werden, wobei in diesem Zusammenhang zweckmäßigerweise ein Gitternetz mit Gitternetzelementen verwendet wird. In vorteilhafter Weise können hierbei die Teilelemente als Polygone, Splines oder andere geeignete mathematische Formulierungen ausgebildet sein.

Nach einer weiteren Ausgestaltung der Erfindung berücksichtigt der maschinenlernende Algorithmus für die Simulation der Verformung des Hohlorgans Steifigkeitsparameter zwischen Teilelementen. Die Steifigkeitsparameter werden als parametrisierte Funktionen der Gewebeeigenschaften des umgebenden Gewebes der jeweiligen Teilelemente aus der medizinischen 3D-Aufnahme entnommen. Als Steifigkeitsparameter werden hierbei Parameter bezeichnet, welche ein Maß für die Unverformbarkeit von Materialien darstellen. Die geeigneten Steifigkeitsparameter werden im Rahmen des Vortrainings ermittelt.

Nach einer weiteren Ausgestaltung der Erfindung werden im Rahmen des Vortrainings diejenigen Steifigkeitsparameter ermittelt, welche eine Minimierung des Fehlers zwischen realer Verformung (also der aus einer tatsächlich aufgenommenen 3D-Aufnahme eines durch das medizinische Instrument verformten Hohlorgans und seines umgebenden Gewebes) und Verformungssimulation bewirken. Durch diese Fehlerminimierung, die mittels der Aufnahme-Paare aus einer ersten medizinischen 3D-Aufnahme eines unverformten Hohlorgans und seines umgebenden Gewebes und einer zweiten medizinischen 3D-Aufnahme eines durch ein medizinisches Instrument verformten Hohlorgans und seines umgebenden Gewebes ermittelt werden, können diejenigen Steifigkeitsparameter bestimmt werden, welche eine besonders realitätsgetreue Verformungssimulation bewirken.

Nach einer weiteren Ausgestaltung der Erfindung werden die Eigenschaften des das Modell umgebenden Gewebes auf das Modell abgebildet, wobei die Eigenschaften insbesondere das lokale Deformationsverhalten des Hohlorgans beeinflussen. In diesem Zusammenhang können verschiedene Alternativen gewählt werden, welche abhängig vom Modell, dem Hohlorgan und dem umgebenden Gewebe eine hochwertige Verformungssimulation gewährleisten können. So kann nach einer besonders einfach ausführbaren Variante das umgebende Gewebe eines Teilelementes durch einen Vektor vorbestimmter Länge auf das Teilelement abgebildet werden. Nach einer zweiten, besonders exakten Variante kann das umgebende Gewebe eines Teilelementes durch einen halbkugelförmigen Umkreis mit vorbestimmtem Radius auf das Teilelement abgebildet werden.

Nach einer weiteren Ausgestaltung der Erfindung wird angenommen, dass Gewebeeigenschaften durch Hounsfield-Einheiten repräsentiert werden, insbesondere für den Fall, dass die medizinische 3D-Aufnahme eine CT- oder DynaCT-Aufnahme ist. Mit der Hounsfield-Skala wird im Allgemeinen die Abschwächung von Röntgenstrahlung in Gewebe beschrieben, die Werte können dann beispielsweise Gewebearten/ -eigenschaften zugeordnet werden.

Nach einer weiteren Ausgestaltung der Erfindung wird der Algorithmus anhand von Aufnahme-Paaren trainiert, bei denen die zweite medizinische 3D-Aufnahme durch eine zweite, physikalisch motivierte Simulation einer Verformung, z.B. durch FE-Methoden, ersetzt wird. Auf diese Weise kann der Algorithmus trainiert werden, eine derartige Simulation exakt nachzubilden.

Nach einer weiteren Ausgestaltung der Erfindung wird die Verformungssimulation an einer Anzeigevorrichtung angezeigt, so dass sie z.B. von einem Arzt begutachtet werden kann. Der Arzt kann dann anhand der angezeigten Verformungssimulation über den tatsächlichen Eingriff entscheiden und bei Bedarf eine Änderung vornehmen.

Nach einer Ausgestaltung der Erfindung wird die Verformungssimulation mit mindestens einer 2D-Aufnahme, insbesondere einer Live-Durchleuchtungsaufnahme, überlagert. Dies ist insbesondere vorteilhaft, wenn ein geplanter oder erfolgender interventioneller Eingriff unter Röntgenüberwachung stattfindet.

Nach einer weiteren Ausgestaltung der Erfindung können die Informationen über das einzuführende medizinische Instrument einer 2D-Aufnahme, insbesondere einer Durchleuchtungsaufnahme, entnommen werden. Die 2D-Aufnahme kann aus einem Speicher herangezogen oder live aufgenommen werden. Die 2D-Aufnahme kann einer Bildverarbeitung unterzogen und hinsichtlich des Instruments segmentiert werden. Die Informationen werden dann der Verformungssimulation zur Verfügung gestellt. Dieser Vorgang kann auch automatisch durchgeführt werden.

Bei dem einzuführenden medizinischen Instrument kann es sich insbesondere um einen Führungsdraht oder eine Gefäßstütze handeln.

Der maschinenlernende, insbesondere auch deep learning basierte, Algorithmus kann z.B. von einem oder mehreren neuronalen Netzen gebildet werden und z.B. mittels teilweise überwachtem ("semi-supervised") Lernverfahren oder bestärkendem Lernen ("Reinforcement Learning") operieren.

Beansprucht wird außerdem ein System zur Durchführung einer Verformungssimulation eines durch das Einführen eines medizinischen Instruments verformbaren Hohlorgans eines Patienten, aufweisend eine Kommunikationsvorrichtung zur Abfrage von medizinischen 3D-Aufnahmen des Hohlorgans mit umgebendem Gewebe, eine Speichervorrichtung zur Speicherung von medizinischen 3D-Aufnahmen des Hohlorgans mit umgebendem Gewebe, eine Bildverarbeitungsvorrichtung zur Durchführung einer Segmentierung von medizinischen 3D-Aufnahmen des Hohlorgans mit umgebendem Gewebe und zur Ermittlung eines dreidimensionalen Modelles der Oberfläche des Hohlorgans mit umgebendem Gewebe, einen vortrainierten maschinenlernenden Algorithmus, der dazu ausgebildet ist, die durch Einführung des Instruments zu erwartende Verformung des Hohlorgans auf der Basis einer segmentierten medizinischen 3D-Aufnahme zu simulieren, eine Recheneinrichtung mit einem Prozessor zur Ausführung des vortrainierten maschinenlernenden Algorithmus, und eine Anzeigevorrichtung zur Anzeige des modellierten, durch das Instrument verformten Hohlorgans. Das System weist hierbei vorteilhaft ein Angiographie-Röntgengerät zur Aufnahme einer medizinischen 3D-Aufnahme von Hohlorganen mit umgebendem Gewebe auf.

Die Erfindung sowie weitere vorteilhafte Ausgestaltungen gemäß Merkmalen der Unteransprüche werden im Folgenden anhand schematisch dargestellter Ausführungsbeispiele in der Zeichnung näher erläutert, ohne dass dadurch eine Beschränkung der Erfindung auf diese Ausführungsbeispiele erfolgt. Es zeigen:

FIG 1      eine Ansicht eines abdominellen Aortenaneurysmas mit eingebrachtem Stent nach dem Stand der Technik;

FIG 2      eine Ansicht einer Überlagerung einer 3D-Aufnahme eines Hohlorgans mit einem 2D-Bild eines Führungsdrahtes nach dem Stand der Technik;

FIG 3      eine Ansicht einer korrigierten Position des Hohlorgans gemäß bekannten Verfahren;

FIG 4      eine Ansicht eines je nach Steifigkeit unterschiedlich gestrichelten Hohlorgans;

FIG 5      eine Abfolge des erfindungsgemäßen Verfahrens;

FIG 5      eine Ansicht des Einflusses des umgebenden Gewebes auf die Steifigkeitsparameter zwischen zwei Polygonen;

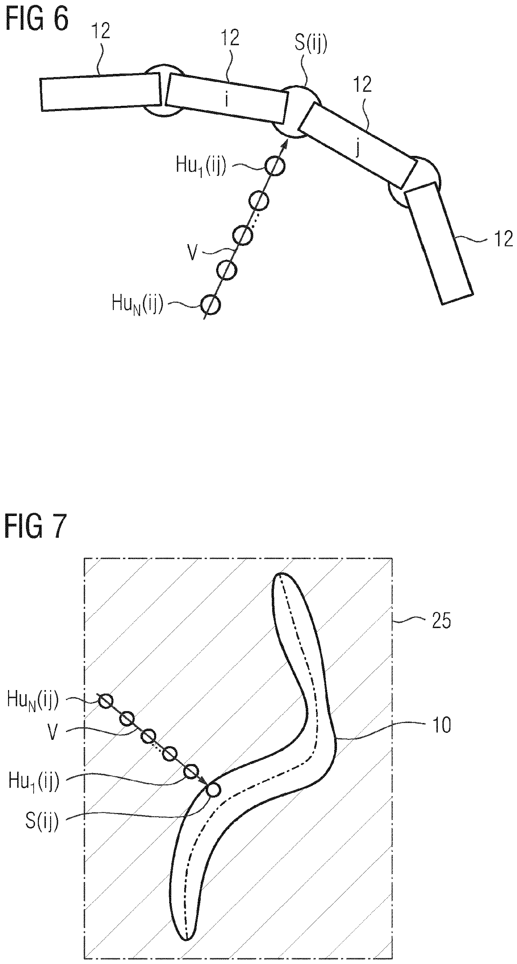

FIG 6      eine Darstellung eines Steifigkeitsparameters und darauf projizierter Hounsfield-Einheiten;

FIG 7      eine 2D-Ansicht einer unverformten Aufnahme mit segmentiertem Hohlorgan und umgebenden Gewebe zur Entnahme von Hounsfield-Einheiten; und

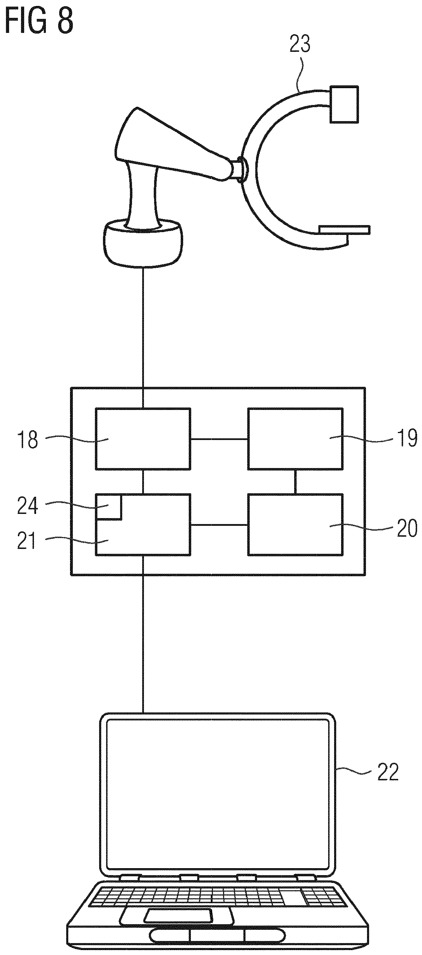

FIG 8      ein System zur Durchführung des erfindungsgemäßen Verfahrens.

In der FIG 5 sind die grundlegenden Schritte des Verfahrens zur Verformungssimulation eines durch das Einführen eines medizinischen Instruments verformbaren Hohlorgans gezeigt. Das Verfahren wird beispielsweise vor dem Start eines interventionellen Eingriffs an dem Hohlorgan durchgeführt, wobei der Eingriff z.B. im Rahmen einer interventionellen Behandlung (z.B. Reparatur) eines Aorten-Aneurysmas durchgeführt werden kann. Das Verfahren kann auch bereits im Operationsraum stattfinden.

In einem ersten Schritt 5 wird ein vortrainierter maschinenlernender Algorithmus, z.B. Deep Learning Algorithmus, bereitgestellt. Verschiedene Möglichkeiten und Einflüsse des Vortrainings des maschinenlernenden Algorithmus werden später detailliert beschrieben. Der maschinenlernende Algorithmus kann z.B. von einem künstlichen neuronalen Netz gebildet werden. Die Verwendung von neuronalen Netzen, welche als Netze aus künstlichen Neuronen aufgebaut sind, ist im Allgemeinen bekannt, siehe z.B. unter https://de.wikipedia.org/wiki/K%C3%BCnstliches_neuronales_Net z.

In einem zweiten Schritt 6 wird zumindest eine medizinische 3D-Aufnahme des Hohlorgans mit seinem umgebenden Gewebe, welche 3D-Aufnahme vor einer Einführung eines medizinischen Instruments aufgenommen wurde, bereitgestellt. Die 3D-Aufnahme kann mittels eines Röntgengerätes, z.B. eines Angiographie-Röntgengerätes oder eines Computertomographen erstellt worden sein. Alternativ kann die 3D-Aufnahme auch direkt angefertigt werden.

In einem dritten Schritt 7 wird die medizinische 3D-Aufnahme des Hohlorgans direkt segmentiert oder in bereits segmentierter Form bereitgestellt. Dabei wird ein dreidimensionales Modell des Hohlorgans ermittelt oder es ist bereits in Form eines Modelles bereitgesellt. Als Modell für das Hohlorgan kann ein Oberflächenmodell oder auch ein Volumenmodell verwendet werden. Insbesondere kann z.B. als Oberflächenmodell ein Polygonnetz mit Netzelementen in Form von Polygonen oder ein Oberflächenmodell basierend auf Splines verwendet werden.

In einem vierten Schritt 8 werden Informationen über das einzuführende medizinische Instrument bereitgestellt. Bei dem Instrument kann es sich zum Beispiel um einen Katheter oder einen Führungsdraht oder eine einzuführende Gefäßstütze handeln. Die Informationen können z.B. aus einer Datenbank/einem Speicher entnommen werden, von einem User eingegeben werden oder aus einer zuvor oder direkt aufgenommenen 2D-Aufnahme (Durchleuchtungsaufnahme) oder 3D-Aufnahme entnommen werden. Die Reihenfolge zwischen dem ersten Schritt 5, dem zweiten Schritt 6 und dem vierten Schritt 8 ist dabei beliebig.

In einem nachfolgenden fünften Schritt 9 wird anschließend mit Hilfe des vortrainierten maschinenlernenden Algorithmus unter Verwendung der segmentierten medizinischen 3D-Aufnahme des Hohlorgans und unter Verwendung von Informationen über das umgebende Gewebe und unter Verwendung der Informationen über das Instrument die durch Einführung des Instruments zu erwartenden Verformung des Hohlorgans simuliert. Dabei wird insbesondere der Einfluss des umgebenden Gewebes auf das jeweilige Verformungsmodell durch einen maschinenlernenden bzw. selbstlernenden Algorithmus bestimmt, um so eine besonders hochwertige Verformungssimulation zu erhalten bzw. eine Verformungskorrektur zu verbessern.

Die Verformungssimulation kann anschließend an einer Anzeigeeinheit 22 angezeigt werden.

Im Folgenden werden zugrundeliegende mathematische Ideen und Details der Verformungssimulation beschrieben. Das dreidimensionale Modell des Hohlorgans mit Teilelementen ist im Folgenden ein Polygonnetz mit Polygonen als Gitternetzelementen. Es können jedoch auch beliebige andere Modelle mit Teilelementen verwendet werden. Die sogenannten Steifigkeitsparameter S(ij) des Modells des Hohlorgans zwischen dem i-ten Polygon i und dem j-ten Polygon j bezeichnen das Maß für die Unverformbarkeit des Hohlorgans - gezeigt in FIG 6. Wird eine Transformationsvorschrift T der Verformungssimulation beschrieben, so ergibt sich

$$MM = T[S, MO],$$

wobei MO die originale unverformte Segmentierung bezeichnet, MM die entsprechende Simulation und S die Gesamtheit der Steifigkeitsparameter S(ij).

Der Einfluss des umgebenden Gewebes auf die Verformung (in der Verformungssimulation also die Steifigkeit der Polygone zueinander) wird basierend auf einer Menge von vorliegenden Daten, sog. Trainingsdaten, gelernt. Als Trainingsdaten dient eine Menge M bekannter Aufnahme-Paare aus jeweils einer ersten medizinischen 3D-Aufnahme eines unverformten Hohlorgans und seines umgebenden Gewebes MO_gt und einer zweiten medizinischen 3D-Aufnahme eines durch ein medizinisches Instrument verformten Hohlorgans und seines umgebenden Gewebes MM_gt. Die Steifigkeitsparameter S(ij) werden hierzu als parametrisierte Funktion des umgebenden Gewebes Gew(ij) formuliert:

$$S(ij) = F\big(W, Gew(ij)\big)$$

Die optimalen Gewichte $W_{opt}$ werden "gelernt" anhand der Menge M bekannter Aufnahme-Paare

$$TM = \big\{\big(MO\_gt(k), MM\_gt(k)\big), k = 1, \dots, M\big\}.$$

Hierbei werden bevorzugt die optimalen Gewichte $W_{opt}$ bestimmt, die eine Minimierung des Fehlers zwischen realer Verformung und simulierter Verformung für die Menge M bewirken. Sind die optimalen Gewichte bestimmt, lässt sich die Verformungssimulation für beliebige 3D-Aufnahmen eines unverformten Hohlorgans und seines umgebenden Gewebes MO berechnen als

$$MM = T\big[F\big(W_{opt}, Gew\big), MO\big].$$

Zur weiteren Implementierung des Verfahrens kann zur konkreten Auswertung der Funktion die Gewebeumgebung Gew(ij) z.B. als diskretes Sampling $Gew(ij) = [G_{1(ij)}, G_{2(ij)}, \dots G_{N(ij)}]$ einer gewissen Umgebung zwischen dem i-ten Polygon i und dem j-ten Polygon j aufgefasst werden, also

$$S(ij) = F\left(W, \left[G_{1(ij)}, G_{2(ij)}, \dots, G_{k(ij)}, \dots G_{N(ij)}\right]\right).$$

Da die für die Verformungssimulation benötigten Polygone (Teilelemente bzw. Gitterelemente) in die 3D-Aufnahme des Hohlorgans und seines umgebenden Gewebes eingebettet sind, wird zur konkreten Berechnung weiter angenommen, dass verschiedene Gewebe(-eigenschaften) durch verschiedene Hounsfield-Einheiten (HU) repräsentiert werden, also dass $G_{k(ij)} = HU_{k(ij)}$ gilt. Unerheblich ist hierbei, dass das gleiche Gewebe ein Spektrum von Hounsfield-Einheiten (HUs) erzeugen kann bzw. verschiedene Gewebe durch den gleichen HU-Wert abgebildet werden können.

Für die Modellierung der Steifigkeitsparameter S(ij) ergibt sich also die (weiterhin unbekannte) Funktion

$$S(ij) = F\left(W, \left[HU_{1(ij)}, HU_{2(ij)}, \dots, HU_{k(ij)}, \dots HU_{N(ij)}\right]\right).$$

In der FIG 6 ist schematisch gezeigt, wie das umgebende Gewebe durch einen Vektor V vorbestimmter Länge auf das Polygon (Gitternetzelement, Teilelement) mit der Steifigkeit S(ij) abgebildet wird, wobei die Gewebeeigenschaften an verschiedenen Punkten 1...N durch Hounsfield-Einheiten $HU_{1(ij)}$... $HU_{N(ij)}$ repräsentiert werden. Alternativ zu dem Vektor V kann das umgebende Gewebe eines Teilelementes z.B. auch durch einen halbkugelförmigen Umkreis mit vorbestimmtem Radius auf das Teilelement abgebildet werden.

Bei einer gegebenen Transformationsvorschrift T zur Verformungssimulation (z.B. ARAP oder eine FEM Modellierung) ergibt sich also für die Transformation insgesamt:

$$MM = T[S, MO] = T[F(W, HU), MO].$$

Die Parameter $W_{opt}$ werden anhand der Minimierung der entsprechenden Fehlerfunktion auf einer Menge von M bekannter Aufnahme-Paare aus jeweils einer ersten medizinischen 3D-Aufnahme eines unverformten Hohlorgans und seines umgebenden Gewebes MO_gt und einer zweiten medizinischen 3D-Aufnahme eines durch ein medizinisches Instrument verformten Hohlorgans und seines umgebenden Gewebes MM gt gelernt

$$W_{opt} = argmin_W \sum_{k=1}^{M} \left\| \left(\overline{MM_{gt(k)}} - T[[W, HU], MO\_gt(k)]\right) \right\|$$

Zur Bestimmung werden entsprechende Lernverfahren verwendet, z.B. Backpropagation mit Gradientenabstiegsverfahren oder andere Methoden zur nichtlinearen Optimierung. Solche Verfahren sind zum Beispiel aus dem Artikel von Pfister et al. Hybrid learning algorithms for neural networks, Zeitschrift für Angewandte Mathematik und Mechanik, Vol. 76, Suppl. 1, 1996, pp. 215ff., bekannt.

Zum Training des maschinenlernenden Algorithmus wird eine ausreichend große Menge von M bekannten Aufnahme-Paaren $TM = \{(MO\_gt(k), MM\_gt(k)), k = 1,...,M\}$ benötigt. Solche Aufnahme-Paare können z.B. aus verformten intraoperativen 3D-Aufnahmen gewonnen werden. Alternativ kann die reale 3D-Aufnahme auch durch eine physikalisch motivierte Simulation, z.B. durch FE-Methoden, ersetzt werden.

Es kann vorkommen, dass die Verformung nicht für alle Polygone aus den zweiten medizinischen 3D-Aufnahmen eines durch ein medizinisches Instrument verformten Hohlorgans und seines umgebenden Gewebes vollständig und genau bekannt ist. In solchen Fällen können die Gewichte auch basierend auf Teilmengen und/oder näherungsweise bekannten Verformungen gelernt werden. Hierbei sind beispielsweise teilweise überwachte ("semisupervised") Lernverfahren oder bestärkendes Lernen ("Reinforcement Learning") denkbar.

Das erfindungsgemäße Verfahren kann bei allen möglichen Anwendungsbereichen für die Adaption von Segmentierungsverformungen von Hohlorganen eingesetzt werden, z.B. in der Neuroradiologie, dem Einsetzen von Aortenklappen oder der Verformung von Gefäßen in der Leber. Es können als 3D-Aufnahmen des Hohlorgans mit umgebendem Gewebe sowohl Röntgenaufnahmen als auch mittels anderer medizinischer Bildgebungsverfahren aufgenommene 3D-Aufnahmen verwendet werden, z.B. MR-Aufnahmen, und entsprechend segmentiert werden. Hier lassen sich die Werte nicht als HU-Werte interpretieren, können aber ansonsten analog verwendet werden.

Die Informationen über das einzuführende medizinische Instrument kann z.B. einer 2D-Aufnahme, insbesondere einer Durchleuchtungsaufnahme, entnommen werden. Die 2D-Aufnahme kann z.B. aus einem Speicher entnommen oder live aufgenommen werden. Die 2D-Aufnahme kann einer Bildverarbeitung unterzogen und hinsichtlich des Instruments segmentiert werden. Die Informationen werden dann der Verformungssimulation zur Verfügung gestellt. Dieser Vorgang kann auch automatisch durchgeführt werden. Bei dem einzuführenden medizinischen Instrument kann es sich insbesondere um einen Führungsdraht oder eine Gefäßstütze handeln.

In der FIG 8 ist ein System zur Durchführung einer Verformungssimulation eines durch das Einführen eines medizinischen Instruments verformbaren Hohlorgans eines Patienten gezeigt. Das System weist eine Recheneinrichtung 21 mit einem Prozessor auf, wobei die Recheneinrichtung 21 dazu ausgebildet ist, einen vortrainierten maschinenlernenden Algorithmus 24, der wiederum dazu ausgebildet ist, die durch Einführung des Instruments zu erwartende Verformung des Hohlorgans auf der Basis einer segmentierten medizinischen 3D-Aufnahme zu simulieren, ausführen kann. Außerdem weist das System eine Kommunikationsvorrichtung 18 zur Abfrage von medizinischen 3D-Aufnahmen des Hohlorgans mit umgebendem Gewebe auf, eine Speichervorrichtung 19 zur Speicherung von medizinischen 3D-Aufnahmen des Hohlorgans mit umgebendem Gewebe und eine Bildverarbeitungsvorrichtung 20 zur Durchführung einer Segmentierung von medizinischen 3D-Aufnahmen des Hohlorgans mit umgebendem Gewebe und zur Ermittlung eines dreidimensionalen Modelles der Oberfläche des Hohlorgans mit umgebendem Gewebe. Außerdem ist dem System eine Anzeigevorrichtung 22 zur Anzeige des modellierten, durch das Instrument verformten Hohlorgans zugeordnet. Das System kann außerdem einem Angiographie-Röntgengerät 23 zur Aufnahme einer medizinischen 3D-Aufnahme von Hohlorganen mit umgebendem Gewebe zugeordnet sein.

Der Vorteil des Verfahrens zur Verformungssimulation unter Verwendung eines maschinenlernenden Algorithmus besteht darin, den Einfluss von Gewebe auf die Verformungssimulation nicht fehlerbehaftet und umständlich experimentell bestimmen zu müssen, wie dies bisher üblich ist, sondern einfach und robust anhand von Beispielen trainieren zu können.

Die Erfindung lässt sich in folgender Weise kurz zusammenfassen: Für eine besonders einfache und gleichzeitig exakte Verformungssimulation eines durch das Einführen eines medizinischen Instruments verformbaren Hohlorgans werden die folgenden Schritte durchgeführt: Bereitstellung eines vortrainierten maschinenlernenden Algorithmus, Bereitstellung einer medizinischen 3D-Aufnahme des Hohlorgans mit umgebendem Gewebe, welche 3D-Aufnahme vor einer Einführung eines medizinischen Instruments aufgenommen wurde, Segmentierung oder Bereitstellung einer Segmentierung der medizinischen 3D-Aufnahme des Hohlorgans und Ermittlung oder Bereitstellung eines dreidimensionalen Modelles des Hohlorgans, Bereitstellung von Informationen über ein einzuführendes medizinisches Instrument, und Simulation der durch Einführung des Instruments zu erwartenden Verformung des Hohlorgans auf der Basis der segmentierten medizinischen 3D-Aufnahme des Hohlorgans und des umgebenden Gewebes und der Informationen über das Instrument durch Verwendung des vortrainierten maschinenlernenden Algorithmus.

**Patentansprüche**

1.  Verfahren zur Verformungssimulation eines durch das Einführen eines medizinischen Instruments verformbaren Hohlorgans (10) mit den folgenden Schritten:

    • Bereitstellung eines vortrainierten maschinenlernenden Algorithmus (24),
    • Bereitstellung einer medizinischen 3D-Aufnahme des Hohlorgans (10) mit umgebendem Gewebe, welche 3D-Aufnahme vor einer Einführung eines medizinischen Instruments (3; 4) aufgenommen wurde,
    • Segmentierung oder Bereitstellung einer Segmentierung der medizinischen 3D-Aufnahme des Hohlorgans (10) und Ermittlung oder Bereitstellung eines dreidimensionalen Modelles des Hohlorgans (10),
    • Bereitstellung von Informationen über ein einzuführendes medizinisches Instrument (3; 4), und
    • Simulation der durch Einführung des Instruments (3; 4) zu erwartenden Verformung des Hohlorgans (10) auf der Basis der segmentierten medizinischen 3D-Aufnahme des Hohlorgans (10) und des umgebenden Gewebes und der Informationen über das Instrument durch Verwendung des vortrainierten maschinenlernenden Algorithmus (24).

2.  Verfahren nach Anspruch 1, wobei das Modell von einem Oberflächen- oder Volumenmodell gebildet wird.

3.  Verfahren nach Anspruch 1 oder 2, wobei der maschinenlernende Algorithmus (24) für die Simulation der Verformung des Hohlorgans Informationen über den Einfluss von Gewebeeigenschaften des umgebenden Gewebes des Hohlorgans (10) berücksichtigt.

4.  Verfahren nach einem der vorangehenden Ansprüche, wobei der maschinenlernende Algorithmus (24) mittels einer Vielzahl von bekannten Aufnahme-Paaren (MO; MM) aus einer ersten medizinischen 3D-Aufnahme eines unverformten Hohlorgans und seines umgebenden Gewebes und einer zweiten medizinischen 3D-Aufnahme eines durch ein medizinisches Instrument (3; 4) verformten Hohlorgans (13) und seines umgebenden Gewebes vortrainiert ist.

5. Verfahren nach Anspruch 4, wobei die Aufnahme-Paare (MO; MM) jeweils bezüglich des Hohlorgans (10) segmentiert sind und das Hohlorgan (10) als dreidimensionales Modell mit Teilelementen, insbesondere als Gitternetz mit Gitternetzelementen dargestellt ist.

6. Verfahren nach Anspruch 5, wobei der maschinenlernende Algorithmus (24) für die Verformungssimulation des Hohlorgans (10) Steifigkeitsparameter ($S_{ij}$) zwischen Teilelementen berücksichtigt, welche Steifigkeitsparameter ($S_{ij}$) als parametrisierte Funktionen der Gewebeeigenschaften des umgebenden Gewebes der jeweiligen Teilelemente aus der medizinischen 3D-Aufnahme entnommen werden.

7. Verfahren nach Anspruch 6, wobei diejenigen Steifigkeitsparameter ($S_{ij}$) ermittelt werden, welche eine Minimierung des Fehlers zwischen realer Verformung und Verformungssimulation bewirken.

8. Verfahren nach einem der vorangehenden Ansprüche, wobei die Eigenschaften des das Modell umgebenden Gewebes auf das Modell abgebildet werden, wobei die Eigenschaften insbesondere das lokale Deformationsverhalten des Hohlorgans (10) beeinflussen.

9. Verfahren nach einem der Ansprüche 5 bis 8, wobei das umgebende Gewebe eines Teilelementes durch einen Vektor (V) vorbestimmter Länge auf das Teilelement abgebildet wird.

10. Verfahren nach einem der Ansprüche 5 bis 8, wobei das umgebende Gewebe eines Teilelementes durch einen halbkugelförmigen Umkreis mit vorbestimmtem Radius auf das Teilelement abgebildet wird.

11. Verfahren nach einem der vorangehenden Ansprüche, wobei angenommen wird, dass Gewebeeigenschaften durch Hounsfield-Einheiten (HU) repräsentiert werden.

12. Verfahren nach einem der Ansprüche 5 bis 11, wobei die Teilelemente als Polygone, Splines oder andere geeignete mathematische Formulierungen ausgebildet sind.

13. Verfahren nach Anspruch 4 oder 5, wobei der Algorithmus (24) anhand der Aufnahme-Paare (MO; MM) trainiert wird, indem die Fehlerfunktion als Fehler zwischen tatsächlicher und simulierter Verformung minimiert wird.

14. Verfahren nach Anspruch 4 oder 5, wobei der Algorithmus anhand der Aufnahme-Paare (MO; MM) trainiert wird, indem die zweite medizinische 3D-Aufnahme durch eine zweite, physikalisch motivierte Simulation, insbesondere durch FE-Methoden, ersetzt wird.

15. Verfahren nach einem der vorangehenden Ansprüche, wobei die Verformungssimulation an einer Anzeigevorrichtung (22) angezeigt wird.

16. Verfahren nach Anspruch 15, wobei die Verformungssimulation mit mindestens einer 2D-Aufnahme, insbesondere einer Live-Durchleuchtungsaufnahme, überlagert wird.

17. Verfahren nach einem der vorangehenden Ansprüche, wobei die Informationen über das einzuführende medizinische Instrument (3; 4) einer 2D-Aufnahme, insbesondere einer Durchleuchtungsaufnahme, entnommen werden.

18. System zur Durchführung einer Verformungssimulation eines durch das Einführen eines medizinischen Instruments (3; 4) verformbaren Hohlorgans (10) eines Patienten nach einem der Ansprüche 1 bis 17, aufweisend

- eine Kommunikationsvorrichtung (18) zur Abfrage von medizinischen 3D-Aufnahmen des Hohlorgans mit umgebendem Gewebe,
- eine Speichervorrichtung (19) zur Speicherung von medizinischen 3D-Aufnahmen des Hohlorgans mit umgebendem Gewebe,
- eine Bildverarbeitungsvorrichtung (20) zur Durchführung einer Segmentierung von medizinischen 3D-Aufnahmen des Hohlorgans (10) mit umgebendem Gewebe und zur Ermittlung eines dreidimensionalen Modelles der Oberfläche des Hohlorgans (10) mit umgebendem Gewebe,
- einen vortrainierten maschinenlernenden Algorithmus (24), der dazu ausgebildet ist, die durch Einführung des Instruments zu erwartende Verformung des Hohlorgans (10) auf der Basis einer segmentierten medizinischen 3D-Aufnahme zu simulieren,
• eine Recheneinrichtung (21) mit einem Prozessor zur Ausführung des vortrainierten maschinenlernenden Algorithmus (24), und
• eine Anzeigevorrichtung (22) zur Anzeige des modellierten, durch das Instrument verformten Hohlorgans (13).

19. System nach Anspruch 18, aufweisend ein Angiographie-röntgengerät (23) zur Aufnahme einer medizinischen 3D-Aufnahme von Hohlorganen mit umgebendem Gewebe.

FIG 1

FIG 2

FIG 3

# FIG 4

# FIG 5

FIG 6

12    12    S(ij)    12

i

$Hu_1(ij)$

V

$Hu_N(ij)$

j

12

FIG 7

$Hu_N(ij)$

V

$Hu_1(ij)$

S(ij)

25

10

FIG 8

Europäisches
Patentamt

European
Patent Office

Office européen
des brevets

# ERKLÄRUNG

die nach Regel 63 des Europäischen Patentübereinkommens für das weitere Verfahren als
europäischer Recherchenbericht gilt

**Nummer der Anmeldung**

EP 19 18 9356

| Die Recherchenabteilung ist der Auffassung, daß die vorliegende Patentanmeldung den Vorschriften des EPÜ in einem solchen Umfang nicht entspricht, daß sinnvolle Ermittlungen über den Stand der Technik auf der Grundlage aller Patentansprüche nicht möglich sind. | **KLASSIFIKATION DER ANMELDUNG (IPC)** |
|---|---|

Grund:

> Die Gründe, für die Erklärung, dass keine Recherche stattgefunden hat, sind in Form 2906 ausführlich beschrieben.
>
> Der Anmelder wird darauf hingewiesen, dass im Zuge der Prüfung eine Recherche durchgeführt werden kann, sollten die einer Erklärung gemäss Regel 63 EPÜ zugrundeliegenden Mängel behoben worden sein (Vgl. EPA-Richtlinien C-IV, 7.2).

INV.
A61B34/10

| Recherchenort | Abschlußdatum | Prüfer |
|---|---|---|
| München | 26. März 2020 | Erbel, Stephan |

EPO FORM 1504 (P04F39)

**IN DER BESCHREIBUNG AUFGEFÜHRTE DOKUMENTE**

**In der Beschreibung aufgeführte Nicht-Patentliteratur**

- **TOTH et al.** Adaption of 3D Models to 2D X-ray Images during Endovascular Abdominal Aneurysm Repair. *Proc. Of the MICCAI Workshop,* 2015, 339-346

- **ROY et al.** Finite element analysis of abdominal aortic aneurysms: geometrical and structural reconstruction with application of an anisotropic material model. *IMA J Appl Math,* 2014, vol. 79 (5), 1011-1026
- **PFISTER et al.** Hybrid learning algorithms for neural networks. *Zeitschrift für Angewandte Mathematik und Mechanik,* 1996, vol. 76, 215ff